# EUROPÄISCHE PATENTSCHRIFT

(11) **EP 0 420 013 B1**
(45) Veröffentlichungstag und Bekanntmachung des Hinweises auf die Patenterteilung: **04.12.1996**
(21) Anmeldenummer: 90117952.3
(22) Anmeldetag: 18.09.1990
(51) Int. Cl.: A61B 5/14

(54) **Blutentnahmevorrichtung**
Means for the withdrawal of blood
Dispositif de prélèvement sanguin

(30) Priorität: 26.09.1989 DE 3932112
(43) Veröffentlichungstag der Anmeldung: 03.04.1991
(73) Patentinhaber: Walter Sarstedt Geräte und Verbrauchsmaterial für Medizin und Wissenschaft, D-51588 Nümbrecht (DE)
(72) Erfinder: Sarstedt, Walter, W-5223 Nümbrecht Rommelsdorf (DE)

(56) Entgegenhaltungen:
- EP-A- 0 058 440
- EP-A- 0 182 370
- DE-A- 3 025 800
- FR-A- 2 230 995

## Beschreibung

Die Erfindung betrifft eine Blutentnahmevorrichtung nach dem Oberbegriff des Patentanspruchs 1.

Es sind bereits kolben- und unterdrucklos arbeitende Blutentnahmevorrichtungen zur Entnahme kleinster Blutmengen bekannt ( DE - PS 27 51 503 ), welche auf der Basis des Kapillareffektes arbeiten und das durch Einstechen einer Wunde in die Fingerkuppe oder das Ohrläppchen des Patienten austretende Blut aufnehmen und in einem Probenröhrchen sammeln können. Nachteilig an diesen Blutentnahmevorrichtungen ist, daß sie mit durch Einstechen einer Wunde austretendem Blut arbeiten müssen, welches mit Gewebeflüssigkeit verunreinigt ist und sich außerdem mit auf der Haut befindlichen Keimen oder Verunreinigungen vermischen kann, so daß keine einwandfreie und dem Venenblut entsprechende Blutprobe vorliegt. Diese Art der Blutentnahme ist auch nicht besonders hygienisch, weil die Haut und eventuell auch die Bekleidung des Patienten mit dem aus der Wunde austretenden Blut verunreinigt werden können.

Aus diesem Grunde hat man bereits Blutentnahmevorrichtungen verwendet, bei denen das rückwärtige Ende einer Kanüle in einem das Blut aufnehmenden Probenröhrchen mündet und die Kanüle in die Vene eines Patienten eingestochen werden kann. Der Blutdruck drückt dann das Blut selbsttätig durch die Kanüle in das Probenröhrchen, wo es gesammelt wird und nach ausreichender Füllung des Probenröhrchens der weiteren Verwendung zugeführt werden kann. Mit derartigen Blutentnahmevorrichtungen wird einwandfreies Venenblut gewonnen, das in keiner Weise durch Keime oder sonstige Fremdkörper verunreinigt ist.

Nachteilig bei diesen bekannten Blutentnahmevorrichtungen ist jedoch, daß im Deckel des Probenröhrchens Entlüftungsbohrungen- bzw. -kanäle vorgesehen sein müssen. Dadurch sind die Manipulationen mit der bekannten Vorrichtung dahingehend eingeschränkt, daß das Probenröhrchen mit seinem hinteren Ende bzw. Boden stets mehr oder weniger nach unten gehalten werden muß, damit das durch die Kanüle in das Probenröhrchen gelangende Blut nicht zu den Entlüftungsöffnungen gelangen und dort austreten kann. Nachteilig ist weiter, daß bei mehr oder weniger nach unten gehaltener Kanüle der Füllvorgang des Probenröhrchen nur schlecht beobachtet werden kann.

Aus der europäischen Offenlegungsschrift EP 0 058 440, von der der Oberbegriff des Anspruchs 1 ausgeht, ist eine Blutentnahmevorrichtung bekannt, bei der sich ein in das Innere eines Probenröhrchen erstreckendes Innenrohr dicht an das hintere Ende einer Kanüle anschließt. Da dieses System durch einen im Röhrchen vorliegenden Unterdruck gefüllt werden soll, ist das vordere Ende des Probenröhrchens hermetisch geschlossen.

Nachteilig bei dieser Blutentnahmevorrichtung ist, daß der Füllvorgang und das Füllvolumen von dem im Probenröhrchen vorherrschenden Unterdruck bestimmt wird. Der während der Herstellung des Röhrchen erzeugte Unterdruck ist aber nicht über einen längeren Zeitraum gleichbleibend, so daß das Volumen des Innenröhrchens nicht exakt gefüllt wird oder aber das Blut bereits bei der Blutentnahme aus dem Innenrohr in das Probenröhrchen gelangt.

Die Aufgabe der Erfindung besteht darin, eine Blutentnahmvorrichtung der eingangs genannten Gattung zu schaffen, bei der das Probenröhrchen und die Kanüle bei der Blutentnahme in jeder beliebigen Position gehalten werden können, ohne daß die Gefahr des Eintretens von Blut in die im allgemeinen im vorderen Ende des Probenröhrchen vorgesehenen Entlüftungskanäle bzw. -öffnungen besteht, wobei außerdem der Füllvorgang genau beobachtet und die Blutentnahme nach Erreichen des gewünschten Füllungsgrades unterbrochen werden kann.

Zur Lösung dieser Aufgabe sieht die Erfindung vor, daß die Entlüftung so ausgebildet ist, daß während der Blutentnahme das Innere des Proberöhrchens zur Atmosphäre entlüftbar ist.

Bevorzugt sollen das Innenrohr und das Probenröhrchen zumindest durchscheinend und vorzugsweise transparent sein.

Der Erfindungsgedanke besteht also darin, daß die Entlüftung so ausgebildet ist, daß während der Blutentnahme das Innere des Probenröhrchens zur Atmosphäre entlüftbar ist. Das Probenröhrchen wird nicht direkt mit dem in die Kanüle eingedrückten Blut gefüllt, sondern zunächst das von außen gut sichtbare Innenrohr. Da das Innenrohr zweckmäßigerweise transparent ist, kann dessen allmähliche Füllung von außen ohne weiteres beobachtet werden und damit die Blutentnahme nach Erreichen des vorbestimmten Füllungsgrads abgebrochen werden. Erst anschließend wird dann das Blut in einer zweiten Phase aus dem Innenrohr in das Probenröhrchen geleitet, innerhalb dessen es dann in jeder gewünschten Weise untersucht oder behandelt werden kann, beispielsweise durch Zentrifugieren oder durch Versand in ein Laboratorium.

Für die Bemessung des Innenrohres ist es zum einen wichtig, daß es ein möglichst großes Volumen aufnehmen kann, weil die durch die Blutentnahmevorrichtung insgesamt aufnehmbare Blutmenge durch das Innenvolumen des Innenrohres begrenzt ist. Andererseits darf das Innenrohr keinen so großen Querschnitt aufweisen, daß beispielsweise bei nach oben gerichteter Kanüle und nach unten gerichtetem Probenröhrchen die bei der Blutentnahme von oben in das Innenrohr gelangende Blutsäule abreißt und somit Blut vorzeitig, d.h. vor Beendigung der Blutentnahme in das Probenröhrchen gelangt. Das Innenrohr weist also einen deutlich größeren Querschnitt als eine Kapillare auf, doch sind die Kapillarkräfte noch ausreichend groß, um einen Zusammenhalt der Blutsäule in jeder Position des Innenrohrs zu gewährleisten.

Vorzugsweise hat das Innenrohr einen kreisförmigen Querschnitt und verläuft koaxial zu dem ebenfalls bevorzugt kreiszylinderförmig ausgebildeten Probenröhrchen.

Besonders vorteilhaft ist es, wenn das Innenrohr und die Kanüle an einem auf das vordere Ende des Probenröhrchens aufgesetzten Deckel angeordnet sind. Dabei soll die Ausbildung insbesondere so sein, daß die Entlüftung im Deckel oder zwischen Deckel und Probenröhrchen oder zwischen Innenrohr und Deckel vorgesehen ist.

Bei dieser Ausführungsform kann bei oder nach der Überführung des Blutes vom Innenrohr in das Probenröhrchen der Deckel mit dem Innenrohr abgenommen werden, so daß schließlich nur noch das in der gewünschten Weise mit Blut gefüllte Probenröhrchen vorliegt, welches dann mit einem Stopfen verschlossen oder aber auch sonstwie für Untersuchungszwecke gehandhabt werden kann.

Sofern das Blut durch Schwerkraft aus dem Innenrohr nach Beendigung der Blutabnahme entfernt werden soll, sieht eine vorteilhafte Ausführungsform der Erfindung vor, daß das Innenrohr einen so großen Innenquerschnitt aufweist, daß bei aus der Vene herausgezogener Kanüle oder wenigstens bei vom vorderen Ende des Innenrohres abgezogener Kanüle und aufrecht stehendem Probenröhrchen die in dem Innenrohr gesammelte Blutsäule selbsttätig nach unten in das Probenröhrchen austritt.

Um bei dieser Ausführungsform eine Verunreinigung des Innenrohres mit in das Probenröhrchen abgegebenem Blut zu verhindern, ist nach einer weiteren vorteilhaften Ausführungsform vorgesehen, daß das Innenrohr nur so weit in das Probenröhrchen hineinragt, daß nach dem Auslaufen der Blutsäule in das Probenröhrchen der Blutspiegel im Probenröhrchen sich unterhalb des unteren Endes des Innenrohres befindet.

Besonders vorteilhaft ist es jedoch, wenn nach der Füllung des Innenrohres mit der Blutsäule ein Unterdruck im Inneren des Probenröhrchens erzeugbar ist, mittels dessen die Blutsäule aus dem Innenrohr in das Probenröhrchen gesaugt wird.

Auf diese Weise wird die in dem Innenrohr befindliche Blutsäule zwangläufig durch den Unterdruck abgesaugt. Obwohl somit auch aus kapillarähnlichen Innenrohren das Blut abgesaugt werden könnte, ist es doch bevorzugt, das Innenrohr mit einem deutlich oberhalb des Querschnittes einer Kapillare liegenden Querschnitt auszustatten, damit ein möglichst großes Blutvolumen vom Innenrohr aufgenommen werden kann.

Wenn das Innenrohr fest im Deckel angeordnet ist, besteht eine erste vorteilhafte Anordnung zur Erzeugung des Unterdrucks nach der Blutentnahme darin, daß der Deckel, der vorzugsweise einen als Zylinder wirkenden Kragen aufweist, mit dem Probenröhrchen, das vorzugsweise einen als Kolben wirkenden Umfangswulst aufweist, nach Art einer Kolben-Zylinder-Anordnung zusammenwirkt, wobei in dem als Zylinder wirkenden Teil wenigstens ein nur in der Blutentnahmeposition die Dichtfläche des den Kolben bildenden Teils überbrückender Entlüftungskanal vorgesehen ist.

Nach einer weiteren vorteilhaften Ausführungsform kann hierzu vorgesehen sein, daß vorzugsweise am hinteren Ende des Innenrohrs ein Kolben angeordnet ist, der mit der Innenwand des Probenröhrchens in Dichtverbindung steht, wobei in der Innenwand des Probenröhrchens wenigstens ein die Dichtfläche des Kolbens nur in der Blutentnahmeposition überbrükkender Entlüftungskanal vorgesehen ist.

Schließlich kann der Unterdruck auf einfache Weise auch dadurch erzeugt werden, daß vorzugsweise am vorderen Ende des Probenröhrchens innen ein Ringkolben angeordnet ist, der mit der Außenwand des Innenrohres in Dichtverbindung steht, wobei in der Außenwand des Innenrohres ein die innere Dichtfläche des Ringkolbens nur in der Blutentnahmeposition überbrückender Entlüftungskanal vorgesehen ist.

Vorteilhafterweise ist das Innenrohr im Deckel axial verschiebbar, d.h. kolbenartig aus der Deckelbohrung herausziehbar, so daß durch Herausziehen des Innenrohres aus dem Deckel der es entleerende Unterdruck erzeugt werden kann. In diesem Fall sollte vorgesehen sein, daß im am weitesten eingeschobenen Zustand das Innenrohr so weit über den Deckel nach außen vorsteht, daß es insbesondere an einer dort vorhandenen geriffelten oder reibenden Oberfläche gut greifbar ist.

Damit auch bei dieser Ausführungsform während der Blutabnahme eine einwandfreie Entlüftung des Innenraumes des Probenröhrchens möglich ist, sieht eine bevorzugte Ausführungsform vor, daß in der Wand des Innenrohres ein im am weitesten eingeschobenen Zustand des Innenrohres die Atmosphäre und den Bereich unmittelbar unterhalb des Deckels miteinander verbindender Entlüftungskanal vorgesehen ist, der zur Unterdruckerzeugung schon bei gerüngfügigem Herausziehen des Innenrohres verschlossen wird, worauf zwischen dem Innenrohr und dem Deckel eine Gleit-Dichtverbindung vorliegt. Besonders einfach kann die Entlüftung dadurch gewährleistet werden, daß der Entlüftungskanal durch eine teilweise Abflächung des Innenrohres gebildet ist, welche im am weitesten eingeschobenen Zustand des Innenrohres sich geringfügig über bzw. unter den Deckel erstreckt.

Um ein ungehindertes Übertreten des Blutes vom Innenrohr in das Probenröhrchen zu gewährleisten, soll bevorzugt weiter vorgesehen sein, daß das Innenrohr im am weitesten eingeschobenen Zustand den Boden des Probenröhrchens noch nicht berührt.

Um die am weitesten eingeschobene Lage des Innenrohres eindeutig zu definieren, ist eine weitere Ausführungsform so ausgebildet, daß ein Anschlag am aus dem Deckel ragenden Teil des Innenrohres die am weitesten eingeschobene Lage des Innenrohres bestimmt.

Damit nach dem Herausziehen des Innenrohres die verbleibende Öffnung im Deckel dicht verschlossen werden kann, soll ein Stopfen zum Verschließen der Deckelbohrung vorgesehen sein, welcher zweckmäßigerweise über ein flexibles Band am Deckel unverlierbar angebracht ist.

Die Erfindung wird im folgenden beispielsweise anhand der Zeichnung beschrieben; in dieser zeigt:
- Fig. 1: eine teilweise geschnittene schematische Seitenansicht einer ersten Ausführungsform einer erfindungsgemäßen Blutentnahmevorrichtung,
- Fig. 1a: die Einzelheit Ia von Fig. 1 in größerem Maßstab,
- Fig. 2: eine entsprechende teilweise geschnittene schematische Seitenansicht einer weiteren Ausführungsform,
- Fig. 3: eine teilweise geschnittene Seitenansicht einer dritten Ausführungsform,
- Fig. 3a: die Einzelheit IIIa von Fig. 3 in vergrößertem Maßstab und
- Fig. 3b: einen Schnitt nach Linie IIIb-IIIb in Fig. 3.

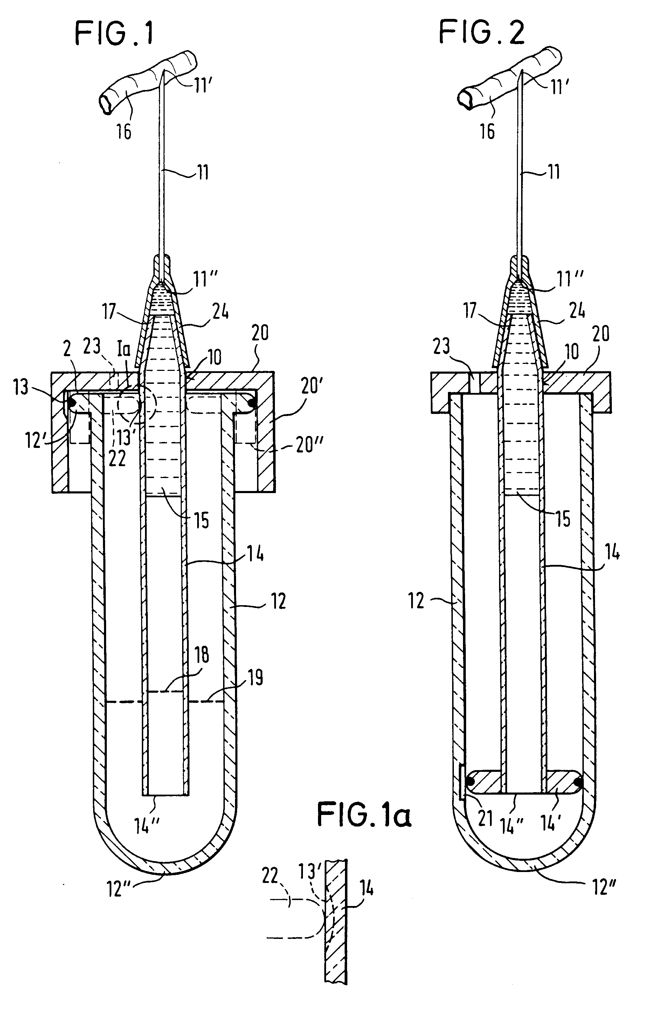

Nach Fig. 1 befindet sich das vordere Ende 11′ einer Kanüle 11 innerhalb einer schematisch angedeuteten Vene 16 eines Patienten. Der hintere Endbereich der Kanüle 11 durchgreift einen Aufsteckkonus 24, der auf einen am vorderen Ende eines Innenrohres 14 ausgebildeten Anbringungskonus 17 dicht aufgesteckt ist. Das hintere Ende 11˝ der Kanüle mündet bündig zum Inneren des Aufsteckkonus 24 hin.

Das Innenrohr 14 erstreckt sich durch eine zentrale Bohrung 10 in einem Deckel 20 hindurch in das Innere eines durch den Deckel 20 oben verschlossenen Probenröhrchens 12, das unten durch einen abgerundeten Boden 12˝ verschlossen ist. Zur Schaffung eines möglichst großen Innenvolumens reicht das Innenrohr 14 bevorzugt bis in den Bereich des Bodens 12˝ des Probenröhrchens 12. Das vordere bzw. obere Ende des Probenröhrchens 12 weist einen Umfangswulst 12′ auf, der nach Art eines Kolbens mit einem nach hinten bzw. unten vorstehenden kreiszylindrischen, radial außen am Deckel 20 angeformten Kragen 20′ zusammenwirkt. In der aus Fig. 1 ersichtlichen Blutentnahmeposition befindet sich der Deckel 20 in der voll auf das Probenröhrchen 12 aufgesetzten Blutentnahmeposition, in welcher ein innen im Kragen 20′ vorgesehener axialer Entlüftungskanal 13 die Umfangsdichtfläche des kolbenartigen Umfangswulstes 12′ überbrückt. Auf diese Weise kann das Innere des Probenröhrchens 12 über den Spalt 2 zwischen dem Deckel 20 und dem Umfangswulst 12′ sowie den Entlüftungskanal 13 entlüftet werden.

Die Arbeitsweise der beschriebenen Blutentnahmevorrichtung ist wie folgt:

Durch den in der Vene 16 vorhandenen Blutdruck wird das Blut in die Kanüle 11 und von dieser durch den Anbringungskonus 17 in das Innenrohr 14 gedrückt, wo sich eine durchgehende Blutsäule 15 bildet, die auch in der in Fig. 1 wiedergegebenen Vertikalposition der Kanüle 11 nicht abreißt, weil der Querschnitt des Innenrohres 14 entsprechend klein dimensioniert ist. Der Querschnitt soll jedoch andererseits möglichst groß sein, damit die dort gesammelte Blutmenge ausreicht, um das Probenröhrchen 12 im späteren Stadium des Füllvorganges möglichst weit zu füllen.

Bevorzugt wird die Blutentnahme soweit fortgesetzt, bis sich die Blutsäule 15 nahe dem hinteren bzw. unteren Ende 14˝ des Innenrohrs 14 befindet. Der Augenblick, wo dieser Füllungsgrad erreicht ist, läßt sich bei jeder beliebigen Stellung des Probenröhrchens 12 bzw. der Kanüle 11 problemlos beobachten, weil sowohl das Probenröhrchen 12 als auch das Innenrohr 14 transparent sind. Nach vollständiger Füllung des Innenrohres 14 wird die Kanüle 11 aus der Vene 16 herausgezogen.

Nunmehr wird der Deckel 20 nach vorn vom Probenröhrchen 12 abgezogen, wobei sich zunächst der Entlüftungskanal 13, der axial entsprechend kurz ausgebildet ist und nur geringfügig unter den Umfangswulst 12 reicht, schließt und anschließend durch die nach Art einer Kolben-Zylinder-Anordnung wirkenden Teile 12′, 20′ ein Unterdruck im Innern des Probenröhrchens 12 aufgebaut wird, der dafür sorgt, daß die Blutsäule 15 aus dem Innern des Innenrohres 14 in das umgebende Probenröhrchen 12 abgesaugt wird. Um diesen Vorgang zu beschleunigen, kann zuvor die Kanüle 11 vom Anbringungskonus 17 abgenommen werden.

Die Länge des Kragens 20′ ist so zu bemessen, daß nach dem Außer-Eingriff-Kommen mit dem Umfangswulst 12′ das Innenrohr 14 vollständig in das Probenröhrchen 12 entleert ist.

Statt des Ringkolben 14′ am unteren Ende des Innenrohres 14 kann in der in Fig. 1 und 1a gestrichelt dargestellten Weise auch am oberen Ende des Probenröhrchens 12 ein innerer Ringkolben 22 vorgesehen sein, der mit der Außenfläche des Innenrohres 14 in Dichtverbindung steht, wobei der Kragen 20˝ unmittelbar an der Außenwand des Probenröhrchens 12 anliegt. Zwischen dem Deckel 20 und dem Innenrohr 14 ist ein den Kanal 13 des ersten Ausführungsbeispiels entsprechender Entlüftungskanal 13′ am Innenrohr 14 vorgesehen, der nur in der aus Fig. 1 und 1a ersichtlichen Blutentnahmeposition die Entlüftung zur Atmosphäre sicherstellt, sofern auch noch die gestrichelt dargestellte Entlüftungsbohrung 23 oder eine andere Deckelentlüftung vorgesehen ist.

Das Verhältnis des Innenvolumens des Innenrohres 14 und des Probenröhrchens 12 soll so gewählt sein, daß die Blutsäule im Probenröhrchen 12 langsamer ansteigt, als das Innenrohr 14 aus dem Probenröhrchen 12 entfernt wird, so daß die Außenseite des Innenrohres 14 bei der Überführung der Blutsäule 15 in das Probenröhrchen 12 nicht mit Blut beschmutzt wird. Auf diese Weise besteht nach vollständiger Herausnahme des Innenrohres 14 aus dem Probenröhrchen 12 keine Verunreinigungsgefahr.

Die Vorrichtung nach Fig. 1 kann aber auch ohne die Kolben-Zylinder-Anordnung 12′, 20′ arbeiten, wobei entweder der Entlüftungskanal 13 axial bis zum unteren Rand des Kragens 20′ reicht oder eine besondere Entlüftungsöffnung 23 im Deckel 20 vorgesehen ist. Allerdings muß in diesem Fall der Querschnitt des Innenrohres 14 so groß sein, daß nach dem Herausziehen der Kanüle 11 aus der Vene 16 oder zumindest nach dem Abnehmen der Kanüle 11 vom Anbringungskonus 17 die Blutsäule 15 in der in Fig. 1 dargestellten aufrechten Position des Probenröhrchens 12 durch die Schwerkraft selbsttätig nach unten in das Probenröhrchen 12 ausläuft. Um auch in diesem Fall eine Verunreinigung der Außenseite des Innenrohres 14 mit Blut zu vermeiden, sollte das untere Ende des Innenrohres 14 etwas weiter vom unteren Ende 12˝ des Probenröhrchens 12 entfernt sein und beispielsweise an der in Fig. 1 gestrichelt dargestellten Stelle 18 liegen. Das durch Schwerkraft in das Probenröhrchen 12 einströmende Blut der Blutsäule 15 würde dann nur bis zur Höhe 19, die in Fig. 1 gestrichelt angedeutet ist, aufsteigen.

Nach Fig. 2, in der gleiche Bezugszahlen entsprechende Teile wie in Fig. 1 darstellen, ist zur Unterdruckerzeugung nach der Blutabnahme am unteren Ende des Innenrohres 14 ein Ringkolben 14′ vorgesehen, der aus Kunststoff mit am Umfang eingelegter Dichtung oder dort vorgesehenen Dichtlippen besteht und so in dichtender Verbindung mit der Innenwand des Probenröhrchens 12 steht. In der aus Fig. 2 ersichtlichen Blutentnahmeposition, in der sich das Innenrohr 14 in seiner untersten bzw. hintersten Stellung befindet, überbrückt ein axialer Entlüftungskanal 21 die Umfangsdichtfläche des Ringkolbens 14′, so daß die beim Ansteigen der Blutsäule 15 verdrängte Luft in den darüberliegenden Raum und schließlich durch die Entlüftungsbohrung 23 im Deckel 20 entweichen kann. Nach einer vorteilhaften Abwandlung ist der Durchmesser des Innenrohres 14 so groß, daß der Kolben 14′ durch einen in eine Umfangsnut des Innenrohres 14 eingelegten O-Ring, der die Innenwand des Probenröhrchens 12 berührt, gebildet werden kann. Es ist auch möglich, daß das Innenrohr 14 fest mit dem Deckel 20 verbunden, insbesondere mit diesem einstückig ist. Dann können diese Teile als ein Spritzgußstück herstellt werden, auf das dann nur noch der O-Ring aufgebracht, insbesondere aufgeschnappt werden muß.

Der Deckel 20 ist auf das vordere Ende des Probenröhrchens 12 abnehmbar dicht aufgesetzt, z.B. aufgeschraubt.

Sobald das Innenrohr 14 bis zum unteren bzw. hinteren Ende 14˝ mit Blut gefüllt ist, wird die Blutentnahme beendet, und nunmehr wird das axial gleitbar in der Deckelbohrung 10 geführte Innenrohr 14 aus dem Probenröhrchen 12 axial herausgezogen. Wenn der Deckel 20 mit dem Innenrohr 14 fest verbunden ist, wird das Innenrohr 14 am Deckel 20 und mit diesem herausgezogen. Der nach Schließung des Entlüftungskanals 21 dabei entstehende Unterdruck im unteren Teil des Probenröhrchens 12 saugt dabei die Blutsäule 15 aus dem Innenrohr 14 heraus in das Probenröhrchen 12.

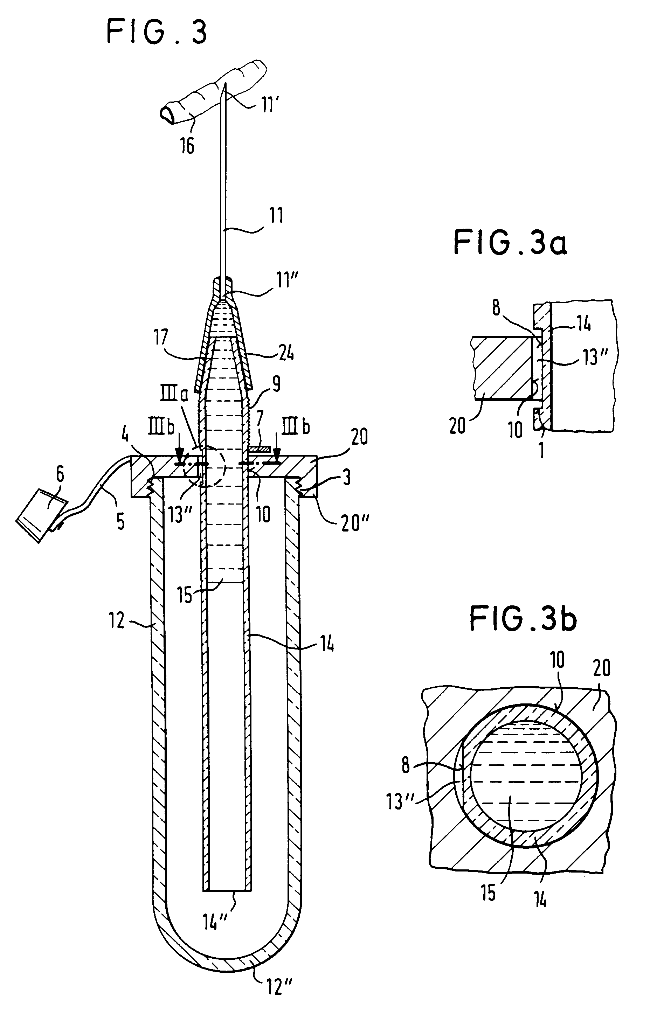

Bei der Ausführungsform nach den Fig. 3, 3a und 3b ist der Deckel 20 mittels des an seinem axial vorstehenden Kragen 20˝ vorgesehenen Innengewindes 3 auf ein Außengewinde 4 am oberen Ende des Probenröhrchens 12 dicht aufgeschraubt. Durch die zentrale Axialbohrung 10 des Deckels 20 ist das kreiszylindrische Innenrohr 14 axial gleitend hindurchgeführt, wobei ein im oberen Bereich vorgesehener Anschlag 7 die Einschubtiefe des Innenrohres 14 in das Probenröhrchen 12 in der aus Fig. 3 ersichtlichen Weise begrenzt.

Gegenüber den vorangehenden Ausführungsbeispielen steht das Innenrohr 14 etwas weiter über den Deckel 20 nach außen vor, wo in dem noch zylindrischen Bereich eine aufgerauhte oder geriffelte Oberfläche 9 vorgesehen ist, an der das Innenrohr 14 zum Zwecke des Herausziehens von der Bedienungsperson kraftschlüssig ergriffen werden kann.

Nach den Fig. 3, 3a und 3b ist die Außenwand des Innenrohres 14 im Bereich der Deckelbohrung 10 derart abgeflacht, daß zwischen den Deckel 20 und dem Innenrohr 14 der insbesondere aus den Fig. 3a und 3b ersichtliche seitliche axiale Entlüftungskanal 13˝ gebildet wird, der sich etwas über und unter den Deckel erstreckt.

Über ein flexibles Band 5, das einstückig mit dem Deckel 20 sein kann, ist ein Stopfen 6 unverlierbar mit dem Deckel 20 verbunden, mittels dessen die Deckelbohrung 10 nach dem Herausziehen des Innenrohres 14 dicht verschlossen werden kann.

Die Arbeitsweise der Ausführungsform nach den Fig. 3, 3a und 3b ist wie folgt:

Vor der Blutabnahme wird das Innenrohr 14 in die Deckelbohrung 10 des auf das Probenröhrchen 12 aufgeschraubten Deckel 20 soweit eingeschoben, bis der Anschlag 7 von oben auf den Deckel 20 aufliegt. In diesem Zustand schafft der Entlüftungskanal 13˝ eine Entlüftungsverbindung zwischen dem Inneren des Probenröhrchens 12 und der umgebenden Atmosphäre.

In diesem Zustand kann Blut 15 abgenommen werden, welches sich ähnlich wie bei den vorangehenden Ausführungsbeispielen als eine Säule 15 allmählich in das Innenrohr 14 ausbreitet, wobei der Durchmesser des Innenrohres 14 und seine Länge so gestaltet sind, daß auch in der vertikalten Position nach Fig. 3 die Blutsäule 15 nicht abreißt und sich wie eine Säule stetig nach unten bewegt. Die Bedienungsperson kann aufgrund der transparenten Ausbildung des Probenröhrchens 12 und des Innenrohres 14 den Füllvorgang gut beobachten und die Blutentnahme bei Erreichen des gewünschten Füllungsgrades unterbrechen. Sobald die Kanüle 11 aus der Vene 16 herausgezogen ist, kann die Bedienungsperson das Innenrohr 14 im aufgerauhten Bereich 9 oberhalb des Deckels 20 ergreifen und das Rohr 14 nach oben herausziehen, wobei zunächst der Entlüftungskanal 13˝ dadurch geschlossen wird, daß seine untere Begrenzung (1 in Fig. 3a) in den Bereich der Deckelbohrung 10 gelangt, von wo ab dann der gesamte Umfang des Innenrohres 14 dichtend am Rand der Deckelbohrung 10 anliegt. Nunmehr wird durch weiteres Herausziehen des Innenrohres 14 ein Vakuum im Inneren des Probenröhrchens 12 erzeugt, aufgrund dessen die Blutsäule 15 aus dem unteren Ende 14˝ des Innenrohres 14 aus- und in das Innere des Probenröhrchens 12 übertritt. Durch die Kanüle 11 kann dabei von der Atmosphäre her Luft nachgesaugt werden, ebenso wie bei den anderen Ausführungsbeispielen.

Bevorzugt wird das Innenrohr 14 vollständig aus dem Deckel 20 herausgezogen und beispielsweise weggeworfen, worauf dann mittels des Stopfens 6, der in die Deckelbohrung 10 dicht von oben eingesetzt wird, das Innere des Probenröhrchens 12 beispielsweise zwecks Versendens des Probenröhrchens 12 hermetisch nach außen abgeschlossen werden kann.

Beim späteren Gebrauch im Labor wird dann der Deckel 20 abgeschraubt, wodurch das Innere des Probenröhrchens 12 zugänglich wird.

Die erfindungsgemäße Blutentnahmevorrichtung ist zweckmäßig für die Aufnahme von 100 bis 1000 µl ausgelegt, wobei der bevorzugte Mengenbereich bei 500 bis 700 µl liegt.

Das Aufnahmevolumen des Innenrohres 14 ist also deutlich größer als das einer Kapillare, die im allgemeinen nur 20 bis 50 µl und äußerstenfalls 100 µl aufnimmt.

Die verwendete Kanüle 11 soll möglichst dünn sein, damit die Füllung des Innenrohres 14 möglichst langsam und übersichtlich vor sich geht.

Maßgebend für die Füllgeschwindigkeit des Innenrohres 14 ist zum einen der vom Benutzer nicht beeinflußbare Venendruck, zum anderen jedoch das Verhältnis zwischen dem Innenquerschnitt der Kanüle 11 und dem Innenquerschnitt des Innenrohres 14. Dieses Verhältnis soll zwischen 1 : 5 und 1 : 15, inbesondere bei 1 : 10 liegen. Bevorzugte Absolutwerte für den Innendurchmesser der Kanüle sind 0,2 bis 0,3, insbesondere 0,25 mm und für den Innendurchmesser des Innenrohres 14 2 bis 5, insbesondere 3 bis 4 und bevorzugt etwa 3,5 mm.

Der Durchmesser des Innenrohres muß unter Berücksichtigung von Kanülendurchmesser und Venendruck so gewählt werden, daß die Fließgeschwindigkeit im Innenrohr so herabgesetzt wird, daß der Füllvorgang genau beobachtet und rechtzeitig abgebrochen werden kann.

Der Deckel 20 kann auf das Probenröhrchen 12 aufgesteckt, aufgeschnappt oder aufgeschraubt sein.

## Patentansprüche

1. Blutentnahmevorrichtung mit einer mit ihrem vorderen Ende ( 11' ) in die Vene ( 16 ) eines Patienten einstechbaren Kanüle ( 11 ), deren hinteres Ende ( 11'' ) in Strömungsverbindung mit dem Inneren eines Probenröhrchens ( 12 ) steht, das an seinem hinteren Ende hermetisch und an seinem vorderen Ende bis auf eine Entlüftung ( 13, 13', 13'' ) geschlossen ist, einem an das hintere Ende ( 11') der Kanüle ( 11 ) sich dicht anschließendes und in das Innere des Probenröhrchens ( 12 ) erstreckendes Innenrohr ( 14 ) von solchem Innenquerschnitt und solcher Länge, daß einerseits ein die Beobachtung und somit das rechtzeitige Abbrechen des Füllvorganges ermöglichendes, möglichst großes Innenvolumen vorliegt und andererseits bei der Blutabnahme vom hinteren Ende ( 11'' ) der Kanüle ( 11 ) in das Innenrohr ( 14 ) eine zusammenhängende Blutsäule gedrückt wird, die bei jeder Position des Probenröhrchens ( 12 ) relativ zur Richtung der Schwerkraft, d.h. auch bei Anordnung unterhalb der Kanüle ( 12 ), zumindest solange nicht abreißt, wie die Kanüle ( 11 ) in der Vene ( 16 ) steckt,
dadurch gekennzeichnet,
daß die Entlüftung so ausgebildet ist, daß während der Blutentnahme das Innere des Probenröhrchens zur Atmosphäre entlüftbar ist.

2. Vorrichtung nach Anspruch 1,
dadurch **gekennzeichnet,**
daß das Innenrohr (14) einen auf das vordere Ende des Probenröhrchens (12) aufgesetzten Deckel (20) durchsetzt und sich vorzugsweise mittig durch eine Bohrung (10) in der Deckelstirnwand (20˝) hindurcherstreckt, wobei das einen Anbringungskonus (17) tragende Ende des Innenrohres (14) über den Deckel (20) nach vorn vorsteht und wobei insbesondere das Innenrohr (14) sich bis in die Nähe des Bodens (12˝) des Probenröhrchens (12) erstreckt, wobei insbesondere die Entlüftung (13, 13′, 13˝) im Deckel (20) oder zwischen dem Deckel (20) und dem Probenröhrchen (12) oder zwischen dem Innenrohr (14) und dem Deckel (20) vorgesehen ist.

3. Vorrichtung nach einem der vorhergehenden Ansprüche,
dadurch **gekennzeichnet,**
daß das Innenrohr (14) einen so großen Innenquerschnitt aufweist, daß bei aus der Vene (16) herausgezogener Kanüle (11) oder bei vom vorderen Ende (17) des Innenrohres (14) abgezogener Kanüle (11) und aufrechtstehendem Probenröhrchen (12) die in dem Innenrohr (14) gesammelte Blutsäule (15) selbsttätig nach unten in das Probenröhrchen (12) austritt, wobei vorzugsweise das Innenrohr (14) nur so weit in das Probenröhrchen (12) hineinragt, daß nach dem Auslaufen der Blutsäule (15) in das Probenröhrchen (12) der Blutspiegel (19) im Probenröhrchen sich unterhalb des unteren Endes (18) des Innenrohres (14) befindet.

4. Vorrichtung nach einem der vorhergehenden Ansprüche,
dadurch **gekennzeichnet,**
daß nach der Füllung des Innenrohres (14) mit der Blutsäule (15) ein Unterdruck im Inneren des Probenröhrchens (12) erzeugbar ist, mittels dessen die Blutsäule (15) aus dem Innenrohr (14) in das Probenröhrchen (12) gesaugt wird, und zwar auch dann, wenn die Schwerkraft hierfür nicht ausreicht, daß bevorzugt das Innenrohr (14) fest mit dem Deckel (20) verbunden ist und ihn dicht durchsetzt und daß weiter zweckmäßigerweise der Deckel (20), der vorzugsweise einen als Zylinder wirkenden Kragen (20′) besitzt, mit dem Probenröhrchen (12), das vorzugsweise einen als Kolben wirkenden Umfangswulst (12′) aufweist, nach Art einer Kolben-Zylinder-Anordnung zusammenwirkt, wobei in der Innenwand des Kragens (20′) wenigstens ein nur in der Blutentnahmeposition die Dichtfläche des Umfangswulstes (12′) überbrückender Entlüftungskanal (13) vorgesehen ist, oder daß vorzugsweise am vorderen Ende des Probenröhrchens (12) innen ein Ringkolben (22) angeordnet ist, der mit der Außenwand des Innenrohres (14) in Dichtverbindung steht, wobei in der Außenwand des Innenrohres (14) ein die innere Dichtfläche des Ringkolbens (22) nur in der Blutentnahmeposition überbrückender Entlüftungskanal (13′) vorgesehen ist, oder daß bevorzugt das Innenrohr (14) in der Deckelbohrung (10) axial verschiebbar ist und im am weitesten eingeschobenen Zustand vorzugsweise noch soweit über den Deckel (20) nach außen vorsteht, daß es insbesondere an einer dort vorhandenen geriffelten oder reibenden Oberfläche (9) gut greifbar ist.

5. Vorrichtung nach Anspruch 4,
dadurch **gekennzeichnet,**
daß vorzugsweise am hinteren Ende des Innenrohres (14) ein Kolben (14′) angeordnet ist, der mit der Innenwand des Probenröhrchens (12) in Dichtverbindung steht, wobei in der Innenwand des Probenröhrchens (12) wenigstens ein die Dichtfläche des Kolbens (14′) nur in der Blutentnahmeposition überbrückender Entlüftungskanal (21) vorgesehen ist, wobei insbesondere in der Wand des Innenrohres (14) ein im am weitesten eingeschobenen Zustand des Innenrohres (14) die Atmosphäre und der Bereich unmittelbar unterhalb des Deckels (20) miteinander verbindender Entlüftungskanal (13˝) vorgesehen ist, der zur Unterdruckerzeugung schon bei geringfügigem Herausziehen des Innenrohres (14) verschlossen wird, worauf zwischen dem Innenrohr (14) und dem Deckel (20) eine Gleit-Dichtverbindung vorliegt.

6. Vorrichtung nach Anspruch 5,
dadurch **gekennzeichnet,**
daß der Entlüftungskanal (13˝) durch eine teilweise Abflächung (8) des Innenrohres (14) gebildet ist, welche im am weitesten eingeschobenen Zustand des Innenrohres (14) sich geringfügig über bzw. unter den Deckel (20) erstreckt.

7. Vorrichtung nach einem der vorhergehenden Ansprüche,
dadurch **gekennzeichnet,**
daß das Innenrohr (14) im am weitesten eingeschobenen Zustand den Boden (12˝) des Probenröhrchens (12) noch nicht berührt.

8. Vorrichtung nach Anspruch 5 oder 6,
dadurch **gekennzeichnet,**
daß ein Anschlag (7) am aus dem Deckel (20) ragenden Teil des Innenrohres (14) die am weitesten eingeschobene Lage des Innenrohres (14) bestimmt.

9. Vorrichtung nach einem der Ansprüche 5 bis 8,
dadurch **gekennzeichnet,**
daß ein Stopfen (6) zum Verschließen der Deckelbohrung (10) vorgesehen ist.

10. Vorrichtung nach einem der vorhergehenden Ansprüche,
dadurch **gekennzeichnet,**
daß das Innenrohr (14) und das Probenröhrchen (12) zumindest durchscheinend und vorzugsweise transparent sind.

## Claims

1. Blood removal device having a cannula (11), the front end (11') of which can be inserted into the vein (16) of a patient and the rear end (11'') of which is in flow connection with the inside of a sample tube (12) sealed hermetically at its rear end and closed at its front end except for a vent opening (13, 13', 13''), an inner tube (14) which adjoins the rear end (11') of the cannula (11) in sealing manner and which extends into the inside of the sample tube (12) and which has an inside cross-section and a length such that, on the one hand, there is as large as possible an inner volume permitting observation and thus discontinuance of the filling operation at the correct time and, on the other hand, when blood is removed, a continuous blood column is forced into the inner tube (14) from the rear end (11'') of the cannula (11), which blood column, regardless of the position of the sample tube (12) relative to the direction of gravity, that is to say, even when it is arranged underneath the cannula (12), is not broken off as long as the cannula (11) remains inserted in the vein (16), characterised in that the vent opening is in a form such that the inside of the sample tube can be vented to the atmosphere during the removal of blood.

2. Device according to Claim 1, characterised in that the inner tube (14) penetrates a lid (20) placed on the front end of the sample tube (12) and extends preferably centrally through a bore (10) in the end wall (20'') of the lid, wherein the end of the inner tube (14), carrying an attachment cone (17), projects forwards beyond the lid (20) and wherein, especially, the inner tube (14) extends as far as the vicinity of the base (12'') of the sample tube (12), wherein, especially, the vent opening (13, 13', 13'') is provided in the lid (20) or between the lid (20) and the sample tube (12) or between the inner tube (14) and the lid (20).

3. Device according to either of the preceding claims, characterised in that the inner tube (14) has so large an inner cross-section that, when the cannula (11) is pulled out of the vein (16) or when the cannula (11) is pulled off the front end (17) of the inner tube (14) and the sample tube (12) is standing upright, the blood column (15) collected in the inner tube (14) is discharged automatically downwards into the sample tube (12), wherein the inner tube (14) preferably projects into the sample tube (12) only to the extent that the blood level (19) in the sample tube is below the lower end (18) of the inner tube (14) after the blood column (15) has run out into the sample tube (12).

4. Device according to any one of the preceding claims, characterised in that, after the inner tube (14) has been filled with the blood column (15), a partial vacuum can be produced inside the sample tube (12), by means of which partial vacuum the blood column (15) is sucked out of the inner tube (14) and into the sample tube (12), even when the gravity is insufficient therefor, in that the inner tube (14) is preferably connected securely to the lid (20) and penetrates it in sealing manner, and in that, also advantageously, the lid (20), which preferably has a collar (20') acting as a cylinder, co-operates with the sample tube (12), which preferably has a circumferential bead (12') acting as a plunger, in the manner of a plunger-cylinder arrangement, at least one venting duct (13) which bridges the sealing face of the circumferential bead (12') only in the blood removal position being provided in the inner wall of the collar (20'), or in that an annular plunger (22) is preferably arranged at the front end of and inside the sample tube (12) and is in sealing connection with the outer wall of the inner tube (14), a venting duct (13') which bridges the inner sealing face of the annular plunger (22) only in the blood removal position being provided in the outer wall of the inner tube (14), or in that the inner tube (14) is preferably axially displaceable in the lid bore (10) and, in the most deeply inserted state, preferably still projects outwards beyond the lid (20) to the extent that it can be gripped well, especially at a fluted or rubbing surface (9) present there.

5. Device according to Claim 4, characterised in that a plunger (14') which is in sealing connection with the inner wall of the sample tube (12) is preferably arranged at the rear end of the inner tube (14), at least one venting duct (21) which bridges the sealing face of the plunger (14') only in the blood removal position being provided in the inner wall of the sample tube (12), wherein, especially, a venting duct (13'') which, in the most deeply inserted state of the inner tube (14), connects the atmosphere and the region immediately below the lid (20) to one another is provided in the wall of the inner tube (14) and, in order to produce a partial vacuum, is closed even if the inner tube (14) is pulled out only slightly, whereupon a sliding sealing connection is present between the inner tube (14) and the lid (20).

6. Device according to Claim 5, characterised in that the venting duct (13'') is formed by a partial flattened portion (8) of the inner tube (14), which portion extends slightly above or below the lid (20) when the inner tube (14) is in the most deeply inserted state.

7. Device according to any one of the preceding claims, characterised in that the inner tube (14) does not touch the base (12'') of the sample tube (12) even when in the most deeply inserted state.

8. Device according to Claim 5 or 6, characterised in that a stop (7) on the portion of the inner tube (14) projecting from the lid (20) determines the most deeply inserted position of the inner tube (14).

9. Device according to any one of Claims 5 to 8, characterised in that a stopper (6) is provided for closing the lid bore (10).

10. Device according to any one of the preceding claims, characterised in that the inner tube (14) and the sample tube (12) are at least translucent and are preferably transparent.

## Revendications

1. Dispositif de prélèvement sanguin comprenant une canule (11) dont l'extrémité avant (11') peut être piquée dans la veine (16) d'un patient et dont l'extrémité arrière (11'') communique, en créant une possibilité d'écoulement, avec l'intérieur d'un tube à essais (12) fermé hermétiquement à son extrémité arrière et, excepté un passage de purge d'air (13, 13', 13''), à son extrémité avant, comprenant un tube intérieur (14) qui est relié de manière étanche à l'extrémité arrière (11'') de la canule (11) et pénètre à l'intérieur du tube à essais (12) et dont la section transversale intérieure et la longueur sont prévues, d'une part, pour que le volume intérieur obtenu soit aussi grand que possible et permette d'observer et ainsi d'interrompre à temps l'opération de remplissage et, d'autre part, pour comprimer, lors du prélèvement sanguin de l'extrémité arrière (11'') de la canule (11) vers le tube intérieur (14), une colonne de sang continue qui, quelle que soit la position du tube à essais (12) par rapport au sens de la force de gravité, c'est-à-dire même au-dessous de la canule (12), ne se rompt pas tout au moins tant que la canule (11) est enfoncée dans la veine (16), caractérisé en ce que le passage de purge d'air est conçu de façon que l'intérieur du tube à essais puisse être purgé de son air lors du prélèvement sanguin.

2. Dispositif selon la revendication 1, caractérisé en ce que le tube intérieur (14) traverse un couvercle (20) emmanché sur l'extrémité avant du tube à essais (12) et s'étend de préférence de manière centrée à travers un trou (10) pratiqué au centre de la paroi frontale (20'') du couvercle, l'extrémité du tube intérieur (14) portant un cône d'insertion (17) dépassant du couvercle (20) vers l'avant, et en particulier le tube intérieur (14) s'étendant jusqu'à proximité du fond (12'') du tube à essais (12), en particulier le passage de purge d'air (13, 13', 13'') étant ménagé dans le couvercle (20) ou entre le couvercle (20) et le tube à essais (12) ou entre le tube intérieur (14) et le couvercle (20).

3. Dispositif selon l'une des revendications précédentes, caractérisé en ce que le tube intérieur (14) présente une section transversale intérieure d'une dimension telle que, lorsque la canule (11) est sortie de la veine (16) ou lorsque la canule (11) est retirée de l'extrémité avant (17) du tube intérieur (14) et que le tube à essais (12) est posé verticalement, la colonne de sang (15) collectée dans le tube intérieur (14) s'échappe spontanément vers le bas dans le tube à essais (12), la longueur de pénétration du tube intérieur (14) dans le tube à essais (12) étant calculée de préférence pour que, une fois la colonne de sang (15) écoulée dans le tube à essais (12), le niveau du sang (19) dans le tube à essais se trouve au-dessous de l'extrémité inférieure (18) du tube intérieur (14).

4. Dispositif selon l'une des revendications précédentes, caractérisé en ce que, après le remplissage du tube intérieur (14) avec la colonne de sang (15), une dépression peut être créée à l'intérieur du tube à essais (12) pour transférer par aspiration la colonne de sang (15) du tube intérieur (14) au tube à essais (12) et ce, même si la force de gravité n'est pas suffisante pour ce transfert, en ce que de préférence le tube intérieur (14) est solidaire du couvercle (20) et le traverse de manière étanche, et en ce qu'en outre, de manière appropriée, le couvercle (20), pourvu de préférence d'un collet (20') faisant fonction de cylindre, coopère à la façon d'un agencement à piston et cylindre avec le tube à essais (12) muni de préférence d'un bourrelet périphérique (12') faisant fonction de piston, dans la paroi intérieure du collet (20') étant ménagé au moins un canal de purge d'air (13) qui ne coïncide avec la surface d'étanchéité du bourrelet périphérique (12') qu'en position de prélèvement sanguin, ou en ce que de préférence à l'extrémité avant du tube à essais (12) est disposé à l'intérieur un piston annulaire (22) qui crée une liaison étanche avec la paroi extérieure du tube intérieur (14), dans la paroi extérieure du tube intérieur (14) étant prévu un canal de purge d'air (13') qui ne coïncide avec la surface intérieure d'étanchéité du piston annulaire (22) qu'en position de prélèvement sanguin, ou en ce que de préférence le tube intérieur (14) peut coulisser axialement dans le trou (10) du couvercle et, dans la position rentrée au maximum, il dépasse encore du couvercle (20) vers l'extérieur de manière à pouvoir être saisi facilement, en particulier au niveau d'une surface rainurée ou antidérapante (9) prévue à cet endroit.

5. Dispositif selon la revendication 4, caractérisé en ce qu'il est prévu, de préférence à l'extrémité arrière du tube intérieur (14), un piston (14') qui crée une liaison étanche avec la paroi intérieure du tube à essais (12), dans la paroi intérieure du tube à essais (12) étant ménagé au moins un canal de purge d'air (21) qui ne coïncide avec la surface d'étanchéité du piston (14') qu'en position de prélèvement sanguin, en particulier dans la paroi du tube intérieur (14) étant prévu un canal de purge d'air (13'') qui, lorsque le tube intérieur (14) se trouve dans la position rentrée au maximum, fait communiquer l'atmosphère et la zone située juste au-dessous du couvercle (20) et qui, pour créer une dépression, est fermé dès que le tube intérieur (14) est légèrement sorti, ce qui crée une liaison étanche par glissement entre le tube intérieur (14) et le couvercle (20).

6. Dispositif selon la revendication 5, caractérisé en ce que le canal de purge d'air (13'') est formé par une zone partiellement aplatie (8) du tube intérieur (14), qui, lorsque le tube intérieur (14) se trouve dans la position rentrée au maximum, s'étend légèrement au-dessus ou au-dessous du couvercle (20).

7. Dispositif selon l'une des revendications précédentes, caractérisé en ce qu'en position rentrée au maximum, le tube intérieur (14) ne touche pas encore le fond (12'') du tube à essais (12).

8. Dispositif selon la revendication 5 ou 6, caractérisé en ce qu'une butée (7) située sur la partie du tube intérieur (14) qui dépasse du couvercle (20) définit la position rentrée au maximum du tube intérieur (14).

9. Dispositif selon l'une des revendications 5 à 8, caractérisé en ce qu'un bouchon (6) est prévu pour obturer le trou du couvercle (10).

10. Dispositif selon l'une des revendications précédentes, caractérisé en ce que le tube intérieur (14) et le tube à essais (12) sont au moins translucides et, de préférence, transparents.
